# EUROPÄISCHE PATENTSCHRIFT

(11) **EP 1 859 744 B1**
(45) Veröffentlichungstag und Bekanntmachung des Hinweises auf die Patenterteilung: **14.12.2011**
(21) Anmeldenummer: 07010303.1
(22) Anmeldetag: 24.05.2007
(51) Int. Cl.: A61B 17/064, A61B 17/08, A61B 17/10, A61B 17/122, A61B 17/00

(54) **Magazin zur Aufnahme von mehreren Clipsen**
Magazine for holding several clips
Magazine destiné à la réception de plusieurs clips

(30) Priorität: 27.05.2006 DE 102006024755
(43) Veröffentlichungstag der Anmeldung: 28.11.2007
(73) Patentinhaber: Aesculap AG, 78532 Tuttlingen (DE)
(72) Erfinder: Mayenberger, Rupert, 78239 Rielasingen (DE); Morales, Pedro, 78532 Tuttlingen (DE)
(74) Vertreter: Hoeger, Stellrecht & Partner Patentanwälte

(56) Entgegenhaltungen:
- WO-A-2006/121490
- US-A- 2 238 736
- US-A- 3 278 107
- US-A- 4 344 531
- US-A- 4 834 098
- US-A- 4 971 198

## Beschreibung

Die Erfindung betrifft ein Magazin von mehreren Clipsen mit einer Entnahmeöffnung zum Einführen eines Clipsanlegewerkzeugs und zur Entnahme eines Clipses aus dem Magazin.

Im chirurgischen Bereich werden zum Verbinden von Gewebeteilen häufig Clipse eingesetzt, die mit einem Clipsanlegewerkzeug aus einem Magazin entnommen und dann an Ort und Stelle durch das Clipsanlegewerkzeug eingesetzt und geschlossen werden. Bekannte Magazine bewahren mehrere Clipse nebeneinander auf, und jedem Clips ist eine eigene Entnahmeöffnung zugeordnet, durch die der im Magazin aufbewahrte Clips entnommen werden kann. Diese Clipsmagazine werden normalerweise auf einer Abstellfläche abgestellt oder an eine Abstellfläche geklebt. Sie sind relativ groß, da für jeden Clips eine Entnahmeöffnung vorgesehen ist, und der Benutzer muss darauf achten, dass das Clipsanlegewerkzeug zur Entnahme eines weiteren Clipses in eine Entnahmeöffnung eingeführt werden muss, aus der bisher noch kein Clips entnommen worden ist, das heißt der Benutzer muss unterscheiden zwischen leeren und vollen Entnahmeöffnungen.

Ebenfalls bekannt sind Magazine mit einer Entnahmeöffnung für die Clipse, welche durch eine Transporteinrichtung nacheinander in die Entnahmeposition verschoben werden (US 497 1198, US 32 78 107).

Es ist Aufgabe der Erfindung, ein gattungsgemäßes Magazin so auszubilden, dass seine Handhabung erleichtert wird.

Diese Aufgabe wird bei einem Magazin der eingangs beschriebenen Art erfindungsgemäß durch ein Magazin gemäß Anspruch 1 gelöst.Das Magazin weist für alle Clipse eine einzige Entnahmeöffnung und eine Transporteinrichtung für die Clipse auf, die die Clipse einzeln nacheinander relativ zur Entnahmeöffnung in eine Entnahmeposition verschiebt, und das Magazin weist eine Außenkontur auf, die es ermöglicht, das Magazin mit einer Hand zu ergreifen, ohne dabei die Entnahmeöffnung abzudecken.

Die Verwendung einer einzigen Entnahmeöffnung erleichtert dem Benutzer die Entnahme, da er nicht mehr zwischen verschiedenen Entnahmeöffnungen auswählen muss und da außerdem sichergestellt ist, dass bei jedem Entnahmevorgang ein Clips zur Verfügung steht, sofern das Magazin nicht vollständig leer ist. Außerdem wird dadurch die Baugröße herabgesetzt, da nur eine einzige Entnahmeöffnung notwendig ist, und dies ermöglicht es, das Magazin grifffreundlich auszubilden, so dass der Benutzer das Magazin in einer Hand halten und mit der anderen Hand das Clipsanlegewerkzeug durch die Entnahmeöffnung einführen kann. Da nur eine einzige Entnahmeöffnung vorgesehen ist, kann die Hand des Benutzers den größten Teil des Magazins umschließen, ohne dass dadurch die Gefahr besteht, dass bestimmte Entnahmeöffnungen ungewollt abgedeckt werden.

Das Magazin weist die Form eines länglichen Zylinders auf und die Entnahmeöffnung ist an einer Stirnseite angeordnet. Ein derartiges Magazin hat die Form einer Trommel oder eines Handgriffs und kann vollständig von der Hand des Benutzers umschlossen werden, wobei die Stirnseite frei bleibt, die die Entnahmeöffnung trägt.

Insbesondere kann der Zylinder ein Kreiszylinder sein.

Es ist günstig, wenn das Magazin auf seiner Außenseite eine Griffprofilierung aufweist, beispielsweise Umfangsrillen zur Aufnahme der Finger des Benutzers, wie dies an sich von Handgriffen bekannt ist.

Vorzugsweise besteht das Magazin zumindest auf seiner Außenseite aus einem grifffreundlichen Material, beispielsweise kann es sich dabei um ein weiches Kunststoffmaterial handeln. Dabei ergibt sich ein angenehmer Griff, und ausserdem kann das Kunststoffmaterial so beschaffen sein, dass es einen hohen Reibungskoeffizienten aufweist, so dass der Benutzer das Magazin sicher halten kann, ohne dass die Gefahr besteht, dass das Magazin aus der Hand heraus gleitet.

Die Länge des Magazins kann beispielsweise zwischen 5 cm und 20 cm liegen, der Durchmesser zwischen 1 cm und 5 cm.

Es ist günstig, wenn die Entnahmeöffnung seitliche Zentrierflächen aufweist, so dass der Benutzer beim Einführen des Clipsanlegewerkzeuges in die Entnahmeöffnung keine genaue Positionierung vornehmen muss, diese Zentrierung und Positionierung des Clipsanlegewerkzeuges wird durch die Zentrierflächen erreicht, die beispielsweise von außen nach innen schräg zusammenlaufen.

Dabei kann insbesondere vorgesehen sein, dass die seitlichen Zentrierflächen einen Abstand voneinander haben, der so bemessen ist, dass Klemmleisten einer Clipsanlegezange bei Anlage an den Zentrierflächen und in vollständig eingeschobenem Zustand einen Abstand voneinander haben, der geringfügig kleiner ist als die Breite der Clipse. Der Benutzer kann daher einfach die Clipsanlegezange so weit öffnen wie möglich, und erhält damit eine Stellung, bei der beim Aufschieben der Klemmbacken auf die Clipse diese geringfügig zusammengedrückt werden, so dass sie im Klemmsitz zwischen den Klemmbacken gehalten werden.

Es kann auch vorgesehen sein, dass statt dessen oder zusätzlich die Clipsanlegezange einen Anschlag aufweist, der den Öffnungszustand der Klemmbacken so begrenzt, dass im vollständig geöffneten Zustand die Klemmbacken einen Abstand voneinander haben, der geringfügig kleiner ist als die Breite der Clipse, so dass auch beim Herausziehen des Clipses aus dem Magazin sichergestellt ist, dass die Clipse zwischen den Klemmbacken im Klemmsitz gehalten werden.

Erfindungsgemäß ist vorgesehen, dass die Clipse in Längsrichtung des Magazins hintereinander angeordnet sind und dass die Transporteinrichtung die Clipse in Längsrichtung des Magazins bei jeder Entnahme eines Clipses um einen Clipsabstand in Richtung auf die Entnahmeöffnung verschiebt.

Die Transporteinrichtung ist durch das Einführen des Clipsanlegewerkzeugs in die Entnahmeöffnung betätigbar. Bei der Entnahme eines Clipses erfolgt damit automatisch ein Nachschieben der übrigen im Magazin angeordneten Clipse in Richtung auf die Entnahmeöffnung, so dass auch beim nächsten Entnahmevorgang ein Clips in der Entnahmeöffnung liegt und von dem Clipsanlegewerkzeug entnommen werden kann.

Erfindungsgemäß ist dabei vorgesehen, dass die Transporteinrichtung einen Schieber umfasst, der beim Einführen des Clipsanlegewerkzeuges von diesem gegen die Wirkung einer Feder parallel zur Längsrichtung des Magazins um eine Clipsabstand in das Magazin einschiebbar ist, und dabei an den im Magazin gehaltenen Clipsen vorbei gleitet, und dass der Schieber beim Herausziehen des Clipsanlegewerkzeugs aus der Entnahmeöffnung unter der Wirkung der Feder wieder in seine Ausgangslage zurückgeschoben wird und dabei alle im Magazin befindlichen Clipse um einen Clipsabstand in Richtung auf die Entnahmeöffnung mitnimmt.

Das Magazin kann zumindest teilweise aus einem durchsichtigen Material bestehen, so dass die Clipse von außen her sichtbar sind.

Dabei ist es insbesondere vorteilhaft, wenn das Magazin zumindest in einem den Clipsen benachbarten durchsichtigen Bereich vergrößerungslinsenartig gekrümmt ist, so dass die Clipse vergrößert betrachtet werden können.

Die nachfolgende Beschreibung bevorzugter Ausführungsformen der Erfindung dient im Zusammenhang mit der Zeichnung der näheren Erläuterung. Es zeigen:

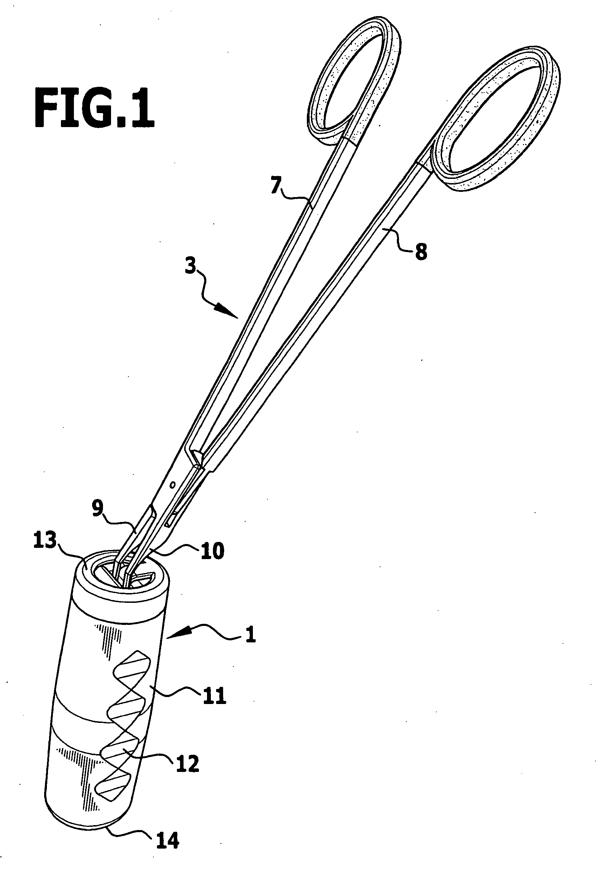

- Figur 1:: eine Ansicht eines trommelförmigen Clipsmagazins mit einer in ihn eingeführten Clipsanlegezange;
- Figur 2:: eine Längsschnittansicht des Magazins der Figur 1 beim Beginn des Einführvorgangs der Clipsanlegezange und
- Figur 3:: eine Ansicht ähnlich Figur 2 bei vollständig eingeschobener Clips-anlegezange.

In einem Magazin 1 werden eine Vielzahl von Clipsen 2 aufbewahrt, die mit Hilfe einer Clipsanlegezange 3 aus dem Magazin 1 entnommen und an der Applikationsstelle, beispielsweise einer zu verschließenden Körperöffnung, angelegt und dort geschlossen werden sollen. Die Clipse haben beispielsweise die Form eines U mit zwei parallelen Schenkeln 4,5 und einem sie verbindenden Steg 6, und sie bestehen aus einem körperfreundlichen Material, beispielsweise aus Titan oder einer Titanlegierung.

Die Clipsanlegezange 3 ist nach Art einer Zange ausgebildet und umfasst zwei schwenkbar aneinander gelagerte Branchen 7, 8 mit gegeneinander verschwenkbaren Klemmleisten 9, 10, die zur Entnahme eines Clipses 2 an die Außenseite der Schenkel 4, 5 eines Clipses 2 angelegt werden. Beim Zusammenschwenken der Klemmleisten 9, 10 wird der Clips dann zusammengedrückt und dadurch geschlossen.

Das die Clipse 2 aufnehmende Magazin ist im Wesentlichen kreiszylindrisch ausgebildet und hat die Form einer Trommel oder eines Handgriffs, wie man ihn von Handgriffen von Schraubenziehern oder Fahrradlenkern kennt. Dabei weist das Magazin 1 ein entsprechend geformtes Gehäuse 11 auf, welches im dargestellten Ausführungsbeispiel aus einem grifffreundlichen, weichen Kunststoffmaterial besteht, beispielsweise aus Tetrapolyethylen oder aus Silikon.

Das Magazin kann aber auch aus einem anderen Material bestehen, beispielsweise aus Polycarbonat, Polystyrol oder einem Acrylnitril-Butadien-StyrolCopolymer, gegebenenfalls mit einer Beschichtung eines grifffreundlichen, weichen Kunststoffmaterials.

An der Außenseite des trommelförmigen Gehäuses 11 sind Vertiefungen 12 angebracht, in die die Finger eines Benutzers eingelegt werden können, wenn das trommelförmige Gehäuse 11 von einem Benutzer so in die Hand genommen wird, dass die Finger das Gehäuse 11 umschließen, wobei eine Stirnseite 13 frei bleibt. Dabei sind die Abmessungen des Gehäuses 11 so gewählt, dass das Gehäuse 11 praktisch vollständig in der Hand des Benutzers aufgenommen wird, die Länge kann beispielsweise in der Größenordnung zwischen 5 cm und 20 cm liegen, der Durchmesser in der Größenordnung zwischen 1 cm und 5 cm.

Im Inneren des Gehäuses 11 ist ein sich durch das gesamte Gehäuse 11 bis zum verschlossenen Boden 14 erstreckender Schacht 15 angeordnet, der an der Stirnseite 13 über eine Entnahmeöffnung 16 mit der Umgebung in Verbindung steht. In diesem Schacht sind hintereinander eine Vielzahl von Clipsen 2 angeordnet, wobei der Abstand zwischen benachbarten Clipsen mindestens so groß gewählt ist wie die Baugröße der Clipse, so dass diese sich nicht überlappen. Alle Clipse werden durch eine Rückhalteleiste 17 gegen ein weiteres Einschieben in das Gehäuse 11 gesichert, und zwar durch Federzungen 18 an der Rückhalteleiste 17, die sich an den Clipsen 12 abstützen. Diese Federzungen 18 können federnd gegen die Rückhalteleiste 17 gebogen werden, so dass die Clipse 2 in Richtung auf die Entnahmeöffnung 16 längs der Rückhalteleiste 17 verschoben werden können und dadurch die Federzungen 18 einbiegen, während eine Verschiebung in der umgekehrten Richtung durch die wieder ausschwenkenden Federzungen 18 vermieden wird.

Die Rückhalteleiste 17 und die Clipse 2 werden konzentrisch umgeben von einem Schieber 19, der mit einem Einführkopf 20 im Bereich der Entnahmeöffnung 16 angeordnet ist und der seitlich an den Clipsen 2 anliegende Verlängerungen 21 trägt, die an ihrer Innenseite den Clipsen zugewandte Vorsprünge 22 aufweisen. Der Schieber 19 ist im Schacht 15 längsverschieblich gelagert und wird durch eine den Schieber 19 konzentrisch umgebende Schraubenfeder 23 gegen die Stirnseite 13 gedrückt.

Die Vorsprünge 22 sind so ausgebildet, dass beim Einschieben des Schiebers 19 entgegen der Wirkung der Schraubenfeder 23 die Vorsprünge 22 an den Clipsen 2 entlang gleiten und diese nicht mitnehmen, da die Clipse durch die Federzungen 18 der Rückhalteleiste 17 gegen ein weiteres Einschieben gesichert sind. Bei Verschiebung des Schiebers 19 in Richtung auf die Entnahmeöffnung 16 dagegen legen sich die Vorsprünge 22 an allen Clipsen 2 an und verschieben diese zusammen mit dem Schieber 19 in Richtung auf die Entnahmeöffnung 16, der Schieber 19 ist somit ein Mitnehmer für die Clipse 2, der diese beim Einschieben unverschoben lässt und sie nur bei der Rückkehr in die ausgeschobene Stellung verschiebt. Die Verschiebebewegung des Schiebers 19 wird dabei durch bauliche Gegebenheiten so begrenzt, dass der Schieber 19 beim Einschieben um mehr als eine Clipsabstand aber um weniger als zwei Clipsabstände verschoben werden kann, die Vorsprünge 22 gleiten dabei jeweils nur über einen Clips hinweg und legen sich bei der Rückbewegung an diesen einen Clips an.

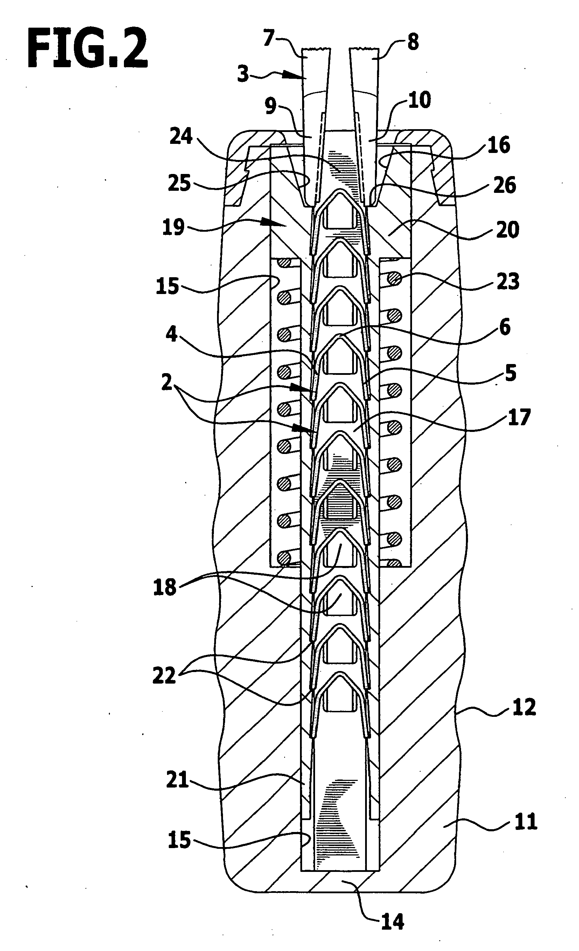

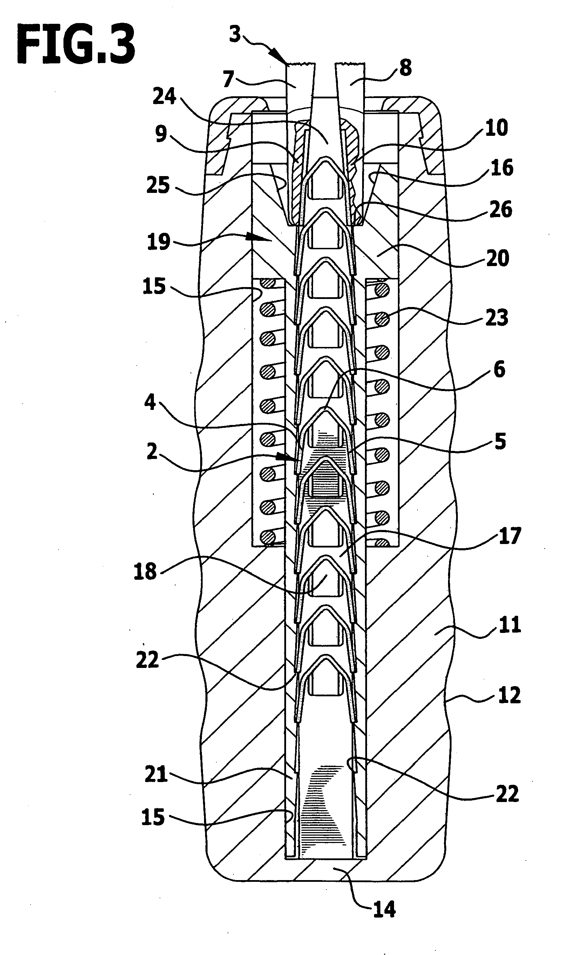

Die Verschiebung des Schiebers 19 erfolgt mittels der Klemmleisten 9, 10 der Clipsanlegezange 3. Dazu weist der Einführkopf 20 eine trichterförmige Einführöffnung 24 mit seitlichen, in Einschieberichtung konvergierenden Zentrierflächen 25 auf. Die Zentrierflächen 25 enden an der tiefsten Stelle der Einführöffnung 24 in einer nach innen vorspringenden Stufe 26, die seitlich neben dem obersten, der Entnahmeöffnung 16 benachbarten Clips 2 liegt und an der sich die Klemmleisten 9, 10 der Clipsanlegezange 3 beim Einführen in die Einführöffnung 24 anlegen (Figur 2). Beim weiteren Einschieben der Klemmleisten 9, 10 in das Gehäuse 11 wird dadurch der Schieber 19 in das Gehäuse 11 eingeschoben, bis die Einschubbewegung beispielsweise durch Anschlag der Verlängerungen 21 am Boden 14 begrenzt wird. Dabei gelangen die Klemmleisten 9, 10 neben den obersten Clips 2 an die Außenseite der Schenkel 4, 5 zur Anlage und nehmen somit diesen obersten Clips 2 zwischen sich auf (Figur 3). Durch leichtes Schließen der Clipsanlegezange 3 kann der Benutzer diesen obersten Clips 2 somit mit der Clipsanlegezange 3 ergreifen und aus dem Gehäuse 11 herausziehen. Dabei verschiebt sich auch der Schieber 19 unter der Wirkung der Schraubenfeder 23 in die ausgeschobene Stellung und nimmt dabei in der beschriebenen Weise alle in dem Gehäuse 11 angeordneten Clipse 2 mit, und zwar um genau einen Clipsabstand, so dass nunmehr der der Entnahmeöffnung 16 benachbarte oberste Clips 2 wieder in einer Entnahmeposition liegt.

Der Entnahmevorgang kann dann wiederholt werden, bis das Magazin 1 leer ist.

## Patentansprüche

1. Magazin (1) zur Aufnahme von mehreren Clipsen (2) in Form eines länglichen Zylinders mit einer einzigen Entnahmeöffnung (16) an einer Stirnseite (13) des Zylinders, wobei das Magazin (1) eine Außenkontur aufweist, die es ermöglicht, das Magazin (1) mit einer Hand zu ergreifen, ohne dabei die Entnahmeöffnung (16) abzudecken, wobei die Clipse (2) in Längsrichtung des Magazins (1) hintereinander in diesem angeordnet sind und eine Transporteinrichtung (19, 20, 21) vorgesehen ist, die die Clipse (2) in Längsrichtung des Magazins (1) bei jeder Entnahme eines Clipses (2) um einen Clips in Richtung auf die Entnahmeöffnung (16) verschiebt, **dadurch gekennzeichnet, dass** die Transporteinrichtung (19, 20, 21) durch das Einführen eines Clipsanlegewerkzeugs (3) in die Entnahmeöffnung (16, 24) betätigbar ist, dass die Transporteinrichtung einen Schieber (19) umfasst, der beim Einführen des Clipsanlegewerkzeuges (3) von diesem gegen die Wirkung einer Feder (23) parallel zur Längsrichtung des Magazins (1) um einen Clipsabstand in das Magazin (1) einschiebbar ist und dabei an den im Magazin (1) gehaltenen Clipsen (2) vorbei geleitet, und dass der Schieber (19) beim Herausziehen des Clipsanlegewerkzeugs (3) aus der Entnahmeöffnung (16, 24) unter der Wirkung der Feder (23) wieder in seine Ausgangslage zurückgeschoben wird und dabei alle im Magazin (1) befindlichen Clipse (2) um einen Clipsabstand in Richtung auf die Entnahmeöffnung (16) mitnimmt.

2. Magazin nach Anspruch 1, **dadurch gekennzeichnet, dass** der Zylinder ein Kreiszylinder ist.

3. Magazin nach einem der voranstehenden Ansprüche, **dadurch gekennzeichnet, dass** das Magazin (1) auf seiner Außenseite eine Griffprofilierung (12) aufweist.

4. Magazin nach einem der voranstehenden Ansprüche, **dadurch gekennzeichnet, dass** das Magazin (1) zumindest auf seiner Außenseite aus einem grifffreundlichen Material besteht.

5. Magazin nach Anspruch 4, **dadurch gekennzeichnet, dass** das grifffreundliche Material ein weiches Kunststoffmaterial ist.

6. Magazin nach einem der voranstehenden Ansprüche, **dadurch gekennzeichnet, dass** die Länge des Magazins (1) zwischen 5 cm und 20 cm liegt.

7. Magazin nach einem der voranstehenden Ansprüche, **dadurch gekennzeichnet, dass** der Durchmesser des Magazins (1) zwischen 1 cm und 5 cm liegt.

8. Magazin nach einem der voranstehenden Ansprüche, **dadurch gekennzeichnet, dass** die Entnahmeöffnung (16, 24) seitliche Zentrierflächen (25) aufweist.

9. Magazin nach Anspruch 8, **dadurch gekennzeichnet, dass** die seitlichen Zentrierflächen (25) einen Abstand voneinander haben, der so bemessen ist, dass Klemmleisten (9, 10) einer Clipsanlegezange (3) bei Anlage an den Zentrierflächen (25) und in vollständig eingeschobenem Zustand einen Abstand voneinander haben, der geringfügig kleiner ist als die Breite der Clipse (2).

10. Magazin nach einem der voranstehenden Ansprüche, **dadurch gekennzeichnet, dass** das Magazin (1) zumindest teilweise aus einem durchsichtigen Material besteht.

11. Magazin nach Anspruch 10, **dadurch gekennzeichnet, dass** das Magazin (1) zumindest in einem den Clipsen (2) benachbarten Bereich aus durchsichtigem Material vergrößerungslinsenartig gekrümmt ist.

## Claims

1. Magazine (1) for accommodating a plurality of clips (2) in the form of an elongate cylinder having a single removal opening (16) at an end face (13) of the cylinder, wherein the magazine (1) has an external contour that enables the magazine (1) to be grasped by a hand without covering the removal opening (16), wherein the clips (2) are disposed one behind the other in the magazine (1) in longitudinal direction thereof and a feed device (19, 20, 21) is provided, which, each time a clip (2) is removed, displaces the clips (2) in longitudinal direction of the magazine (1) one clip further towards the removal opening (16), **characterized in that** the feed device (19, 20, 21) is actuable by introducing a clip application instrument (3) into the removal opening (16, 24), that the feed device comprises a slide (19) that, upon introduction of the clip application instrument (3), is pushable thereby counter to the action of a spring (23) parallel to the longitudinal direction of the magazine (1) by the distance of one clip into the magazine (1) and in the process slides past the clips (2) held in the magazine (1), and that the slide (19) upon withdrawal of the clip application instrument (3) from the removal opening (16, 24) is pushed under the action of the spring (23) back into its initial position and in the process carries along all of the clips (2) situated in the magazine (1) by the distance of one clip in the direction of the removal opening (16).

2. Magazine according to claim 1, **characterized in that** the cylinder is a circular cylinder.

3. Magazine according to one of the preceding claims, **characterized in that** the magazine (1) at its exterior has a gripping profile (12).

4. Magazine according to one of the preceding claims, **characterized in that** the magazine (1) at least at its exterior is made of an easy-to-grip material.

5. Magazine according to claim 4, **characterized in that** the easy-to-grip material is a soft plastics material.

6. Magazine according to one of the preceding claims, **characterized in that** the length of the magazine (1) is between 5 cm and 20 cm.

7. Magazine according to one of the preceding claims, **characterized in that** the diameter of the magazine (1) is between 1 cm and 5 cm.

8. Magazine according to one of the preceding claims, **characterized in that** the removal opening (16, 24) has lateral centring surfaces (25).

9. Magazine according to claim 8, **characterized in that** the lateral centring surfaces (25) have a spacing from one another that is so dimensioned that clamping strips (9, 10) of clip application forceps (3) upon abutment with the centring surfaces (25) and in the fully inserted state have a spacing from one another that is slightly smaller than the width of the clips (2).

10. Magazine according to one of the preceding claims, **characterized in that** the magazine (1) is made at least partially from a transparent material.

11. Magazine according to claim 10, **characterized in that** the magazine (1) at least in a region of transparent material that is adjacent to the clips (2) is curved in the manner of a magnifying lens.

## Revendications

1. Etui (1) pour recevoir plusieurs clips (2) et réalisé sous la forme d'un cylindre allongé présentant une seule ouverture de prélèvement (16) sur une face frontale (13) du cylindre, l'étui (1) présentant un contour extérieur, qui permet de saisir l'étui (1) à une main sans recouvrir l'ouverture de prélèvement (16) à cette occasion, les clips (2) étant agencés les uns derrière les autres dans l'étui (1), dans la direction longitudinale de celui-ci, et il est prévu un dispositif de transport (19, 20, 21) qui fait coulisser les clips (2) dans la direction longitudinale de l'étui (1) en direction de l'ouverture de prélèvement (16), d'une valeur d'un clip à chaque prélèvement d'un clip (2), **caractérisé en ce que** le dispositif de transport (19, 20, 21) peut être actionné par l'introduction d'un outil de pose de clips (3) dans l'ouverture de prélèvement (16, 24), **en ce que** le dispositif de transport comprend un coulisseau (19) qui, lors de l'introduction de l'outil de pose de clips (3), peut, sous l'effet de celui-ci, être repoussé par coulissement dans l'étui (1), à l'encontre de l'action d'un ressort (23), parallèlement à la direction longitudinale de l'étui (1), d'une valeur d'un espacement entre clips, en glissant par-delà les clips (2) maintenus dans l'étui (1), et **en ce que** le coulisseau (19), lors du retrait de l'outil de pose de clips (3) hors de l'ouverture de prélèvement (16, 24), est à nouveau repoussé dans sa position initiale sous l'effet de l'action du ressort (23), en entraînant à cette occasion tous les clips (2) se trouvant dans l'étui (1), d'une valeur d'un espacement entre clips, en direction de l'ouverture de prélèvement (16).

2. Etui selon la revendication 1, **caractérisé en ce que** le cylindre est un cylindre circulaire.

3. Etui selon l'une des revendications précédentes, **caractérisé en ce que** l'étui (1) présente sur son côté extérieur, un profilage de préhension (12).

4. Etui selon l'une des revendications précédentes, **caractérisé en ce que** l'étui (1) est constitué, au moins sur son côté extérieur, d'un matériau facilitant la préhension.

5. Etui selon la revendication 4, **caractérisé en ce que** le matériau facilitant la préhension est une matière plastique souple.

6. Etui selon l'une des revendications précédentes, **caractérisé en ce que** la longueur de l'étui (1) se situe entre 5 cm et 20 cm.

7. Etui selon l'une des revendications précédentes, **caractérisé en ce que** le diamètre de l'étui (1) se situe entre 1 cm et 5 cm.

8. Etui selon l'une des revendications précédentes, **caractérisé en ce que** l'ouverture de prélèvement (16, 24) présente des surfaces de centrage latérales (25).

9. Etui selon la revendication 8, **caractérisé en ce que** les surfaces de centrage latérales (25) présentent une distance d'espacement réciproque, qui est dimensionnée de façon telle que des branches de serrage (9, 10) d'une pince de pose de clips (3), lorsqu'elles s'appuient contre les surfaces de centrage (25), et dans l'état totalement inséré, présentent une distance entre elles, qui est légèrement inférieure à la largeur des clips (2).

10. Etui selon l'une des revendications précédentes, **caractérisé en ce que** l'étui (1) est réalisé, au moins en partie, en un matériau transparent.

11. Etui selon la revendication 10, **caractérisé en ce que** l'étui (1) présente, au moins dans une zone en matériau transparent, voisine des clips (2), une courbure à la manière d'une lentille de grossissement.
